# EUROPEAN PATENT APPLICATION

(11) **EP 4 064 286 A1**
(43) Date of publication of application: **28.09.2022**
(21) Application number: 21168676.1
(22) Date of filing: 15.04.2021
(51) Int. Cl.: G16H 10/60, G16H 15/00, G16H 20/10, G16H 40/20, G16H 40/63, G16H 50/30, G16H 80/00, A61B 5/00

(54) **MEDICATION RISK EVALUATION METHOD AND MEDICATION RISK EVALUATION DEVICE**

(30) Priority: 04.03.2021 TW 110107672
(71) Applicant: Acer Incorporated, New Taipei City 221 (TW); National Yang Ming Chiao Tung University, Hsinchu 30010 (TW)
(72) Inventor: Chen, Pei-Jung, 221 New Taipei City (TW); Tsai, Tsung-Hsien, 221 New Taipei City (TW); Chen, Liang-Kung, 30010 Hsinchu (TW); Huang, Shih-Tsung, 10617 Taipei (TW); Hsiao, Fei-Yuan, 10617 Taipei (TW)
(74) Representative: 2K Patentanwälte Blasberg Kewitz & Reichel

(57) **Abstract**

A medication risk evaluation method and a medication risk evaluation device (10) are provided. The medication risk evaluation method includes: obtaining first medication route information related to a first medicine combination in a medication database (101); obtaining second medication route information related to a second medicine combination in the medication database (101); obtaining overlapping medication information between the first medication route information and the second medication route information; determining whether the overlapping medication information meets a noise exclusion condition; and if the overlapping medication information meets the noise exclusion condition, establishing a risk evaluation model (102) based on other medication route information in the medication database (101) excluding the first medication route information, where the risk evaluation model (102) is adapted to evaluate a risk of using at least one medicine in the medication database (101).

## Description

### BACKGROUND

### Technical Field

The disclosure relates to a medication risk evaluation technology, particularly to a medication risk evaluation method and a medication risk evaluation.

### Description of Related Art

Although medicines are used to treat diseases, they may also put patients at health risks, especially when multiple medicines taken continually by a patient may trigger different chain reactions with one another. Therefore, it is one of the research subjects for those skilled in the art to confirm whether different medicines taken continually by the same patient may interfere with one another, and even cause adverse effects on the patient's body.

### SUMMARY

The disclosure provides a medication risk evaluation method and a medication risk evaluation device capable of using screened medication route information to establish a medication risk evaluation model.

An embodiment of the present disclosure provides a medication risk evaluation method, which includes: obtaining first medication route information related to a first medicine combination in a medication database; obtaining second medication route information related to a second medicine combination in the medication database; obtaining overlapping medication information between the first medication route information and the second medication route information; determining whether the overlapping medication information meets a noise exclusion condition; and if the overlapping medication information meets the noise exclusion condition, establishing a risk evaluation model based on other medication route information in the medication database excluding the first medication route information, wherein the risk evaluation model is adapted to evaluate a risk of using at least one medicine in the medication database.

The embodiment of the present disclosure further provides a medication risk evaluation device, which includes a storage circuit and a processor. The storage circuit is adapted to store a medication database. The processor is coupled to the storage circuit. The processor is adapted to: obtain first medication route information related to a first medicine combination in the medication database; obtain second medication route information related to a second medicine combination in the medication database; obtain overlapping medication information between the first medication route information and the second medication route information; determine whether the overlapping medication information meets a noise exclusion condition; and if the overlapping medication information meets the noise exclusion condition, establish a risk evaluation model based on other medication route information in the medication database excluding the first medication route information, wherein the risk evaluation model is adapted to evaluate a risk of using at least one medicine in the medication database.

Based on the above, the medication risk evaluation method and the medication risk evaluation device provided by the embodiments of the present disclosure are capable of excluding the medication route information that may cause noise or uncertainty in the process of establishing, training, or updating the medication risk evaluation model, thereby improving the reliability of the established risk evaluation model.

### BRIEF DESCRIPTION OF THE DRAWINGS

FIG. 1 is a functional block diagram of a medication risk evaluation device according to an embodiment of the present disclosure.
FIG. 2 is a schematic diagram of first users' record of medication history according to an embodiment of the present disclosure.
FIG. 3 is a schematic diagram of the medication route information of the first users according to an embodiment of the present disclosure.
FIG. 4 is a schematic diagram of medication route information of a second user according to an embodiment of the present disclosure.
FIG. 5 is a schematic diagram of information based on first medication route information, second medication route information, and overlapping medication information according to an embodiment of the present disclosure.
FIG. 6 is a schematic diagram of information based on first medication route information, second medication route information, and overlapping medication information according to an embodiment of the present disclosure.
FIG. 7 is a flowchart of a medication risk evaluation method according to an embodiment of the present disclosure.

### DESCRIPTION OF THE EMBODIMENTS

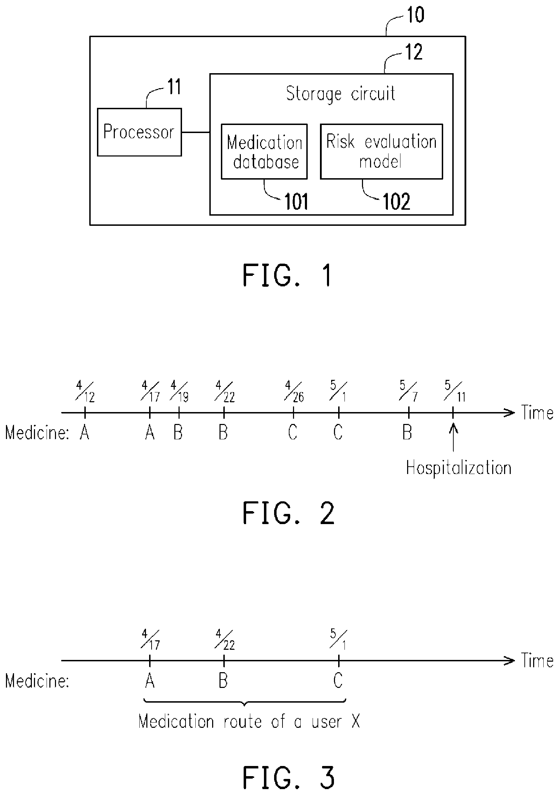

FIG. 1 is a functional block diagram of a medication risk evaluation device according to an embodiment of the present disclosure. In FIG. 1, a device (also referred to as a medication risk evaluation device) 10 may be implemented as a notebook computer, a desktop computer, a tablet computer, an industrial computer, a server, or other types of computer devices.

The medication risk evaluation device 10 includes a processor 11 and a storage circuit 12. The processor 11 is adapted to control the whole or part of the operation of the medication risk evaluation device 10. For example, the processor 11 may include a central processing unit (CPU), or other programmable general-purpose or special-purpose microprocessors, digital signal processors (DSP), programmable controller, application specific integrated circuits (ASIC), programmable logic device (PLD), other similar devices, or a combination thereof.

The storage circuit 12 is coupled to the processor 11 and is adapted to store data. For example, the storage circuit 12 may include a volatile storage circuit and a non-volatile storage circuit. The volatile storage circuit is adapted to store data volatilely. For example, the volatile storage circuit may include random access memory (RAM) or other similar volatile storage media. The non-volatile storage circuit is adapted to store data non-volatilely. For example, the non-volatile storage circuit may include read only memory (ROM), solid state disk (SSD), hard disk drive (HDD), and/or other similar non-volatile storage media.

Note that the medication risk evaluation device 10 may also include various input/output devices, such as a display, a mouse, a keyboard, a touch panel, a touch screen, a speaker, a microphone, a network interface card, and/or a power supply circuit. The present disclosure does not limit the type of the input/output devices.

In one embodiment, the storage circuit 12 stores a medication database 101. The medication database 101 may be adapted to store a record of medication history of one or more users (also referred to as patients). For example, the record of medication history of a certain user may reflect the medication history of the user in a period of time in the past, including the name of the medicine used by the user and the time (for example, the date) when the specific medicine is administrated. In addition, the medication database 101 may also be adapted to store more useful information, which is not limited by the present disclosure.

In one embodiment, the storage circuit 12 also stores a risk evaluation model 102. The risk evaluation model 102 may be adapted to evaluate a risk of using at least one medicine in the medication database 101. In an embodiment, the risk evaluation model 102 may also be adapted to evaluate possible risks of the sequential or combined use of multiple medicines in the medication database 101. For example, after the risk evaluation model 102 is established, the risk evaluation model 102 analyzes the order of administration of these medicines and the possible risks in the use of these medicines. For example, according to the analysis result, the risk evaluation model 102 outputs a report to present the possible risks (for example, possible side effects) of certain medicines in sequential or combined use. In addition, the risk evaluation model 102 may also output more information related to the risk of using the medicines, which is not limited by the present disclosure.

In an embodiment, the risk evaluation model 102 may include a neural network model and/or a machine learning model. In this way, the risk evaluation model 102 may be trained to improve the accuracy of risk evaluation for the use of specific medicines. For example, the processor 11 trains or updates the risk evaluation model 102 with the medication route information related to a specific medicine.

In one embodiment, the processor 11 obtains medication route information (also referred to as first medication route information) related to a certain medicine combination (also referred to as a first medicine combination) in the medication database 101. The first medicine combination includes multiple medicines (also referred to as a plurality of first medicines). In one embodiment, the processor 11 obtains medication route information (also referred to as second medication route information) related to another medicine combination (also referred to as a second medicine combination) in the medication database 101. The second medicine combination also includes multiple medicines (also referred to as a plurality of second medicines).

In one embodiment, the first medication route information reflects the order of use of the medicines in the first medicine combination (i.e., the first medicines). For example, if the first medicine combination includes medicines A, B, and C, the first medication route information reflects a user's order of using the medicines A, B, and C. Similarly, the second medication route information may reflect the order of use of multiple medicines in the second medicine combination (i.e., the second medicine). For example, if the second medicine combination includes medicines D, E, and F, then the second medication route information reflects a user's order of using the medicines D, E, and F.

In one embodiment, the processor 11 obtains overlapping medication information between the first medication route information and the second medication route information. For example, the overlapping medication information reflects a degree of overlap (or an overlap ratio) between the number of medication users that meet the first medication route information and the number of medication users that meet the second medication route information in the medication database 101. In one embodiment, this degree of overlap is represented by a ratio value. For example, according to the record of medication history in the medication database 101, the higher the degree of overlap between the number of medication users that meet the first medication route information and the number of medication users that meet the second medication route information is, the larger the ratio value is.

In one embodiment, after obtaining the overlapping medication information, the processor 11 determines whether the overlapping medication information meets a noise exclusion condition. This noise exclusion condition is adapted to screen out and exclude the information (i.e., noise) that may subsequently have adverse effects on the risk evaluation model 102 (for example, reducing the reliability of the risk evaluation model 102) before establishing the risk evaluation model 102.

In one embodiment, if the processor 11 determines that the obtained overlapping medication information meets the noise exclusion condition, then the processor 11 establishes the risk evaluation model 102 based on other medication route information in the medication database 101 excluding the first medication route information. In other words, before establishing, training, or updating the risk evaluation model 102, the processor 11 may screen out the medication route information that may subsequently have adverse effects on the risk evaluation model 102 according to the noise exclusion condition. And later when establishing, training, or updating the risk evaluation model 102, such medication route information that may have adverse effects on the risk evaluation model 102 is excluded (that is, it is not used to establish, train, or update the risk evaluation model 102), thereby improving the reliability of the risk evaluation model 102 established.

In one embodiment, if the processor 11 determines that the overlapping medication information does not meet the noise exclusion condition, it indicates that the first medication route information is helpful in establishing, training, or updating the risk evaluation model 102 (for example, increasing the amount of information in the risk evaluation model 102). Therefore, in the subsequent process of establishing, training, or updating the risk evaluation model 102, the processor 11 may allow the use of the first medication route information to establish, train, or update the risk evaluation model 102.

In one embodiment, the medicines in the first medicine combination are exactly the same as the medicines in the second medicine combination. In other words, if the first medicine combination includes medicines A, B, and C (or it consists of medicines A, B, and C), the second medicine combination also includes medicines A, B, and C (or it also consists of medicines A, B, and C).

In one embodiment, the medicines in the first medicine combination are completely different from the medicines in the second medicine combination. That is, assuming that the first medicine combination includes medicines A, B, and C (or it consists of medicines A, B, and C), the second medicine combination may include medicines D, E, and F (or it may consist of medicines D, E, and F), where the medicines A, B, and C are completely different from the medicines D, E, and F.

In an embodiment, the processor 11 determines whether the ratio value is greater than a threshold value. If the processor 11 determines that the ratio value is greater than the threshold value, the processor 11 may determine that the overlapping medication information meets the noise exclusion condition. Conversely, if the processor 11 determines that the ratio value is not greater than the threshold value, the processor 11 may determine that the overlapping medication information does not meet the noise exclusion condition.

FIG. 2 is a schematic diagram of first users' record of medication history according to an embodiment of the present disclosure. In FIG. 2, the record of medication history of a user (for example, a user X) reflects that user X took the medicine A on April 12 and April 17 (for example, as he receives the medicine A from the pharmacy), took the medicine B on April 19, April 22, and May 7 (for example, as he receives the medicine B from the pharmacy), and took the medicine C on April 26 and May 1 (for example, as he receives the medicine C from the pharmacy). And later the user X was hospitalized due to a specific illness on May 11. The processor 11 may obtain the medication route information (i.e., the first medication route information) of the user X within a period of time before the hospitalization on May 11 according to the record of medication history of the user X.

FIG. 3 is a schematic diagram of the medication route information of the first users according to an embodiment of the present disclosure. In FIG. 3, which follows the embodiment of FIG. 2, in one embodiment, according to the earliest time of using the medicines and the date when the various medicines are used closest to the hospitalization date, the processor 11 concludes that the medication route of the user X using the medicines A, B, and C is as follows: the user X used the medicines A, B, and C in sequence on April 17, April 22, and May 1. The processor 11 may record this medication route information (i.e., the first medication route information) in the medication database 101.

Note that according to different predetermined rules, the processor 11 may also summarize the different medication routes of the user X using the medicines A, B, and C (including the change in the date of using the medicines and/or the change in the order of using the medicines) based on the record of medication history shown in FIG. 2. For example, in one embodiment, the processor 11 concludes that the medication route of the user X using the medicines A, B, and C is as follows: the user X uses the medicines A, B, and C on April 12, April 19, and April 26 in sequence. Or, in an embodiment, the processor 11 summarizes the medication route of the user X using the medicines A, B, and C as follows: the user X uses the medicines A, B, and C on April 17, May 1, and May 7 in sequence.

In one embodiment, based on the information in the medication database 101, the processor 11 obtains the total number (for example, 50 people) of users (for example, the first users) who have the same medication route (that is, the first medication route) as the user X in FIG. 3. In other words, the first users include every user who used the medicines A, B, and C in sequence. Among them, for each of the first users, the dates on which the medicines A, B, and C are used in sequence may be different. For example, the user X used the medicines A, B, and C on April 17, April 22, and May 1, while a user T used the medicines A, B, and C on January 5, January 18, and January 27. The user T may also be regarded as the first users, who have the same medication route as the user X.

In one embodiment, after obtaining the first users, according to the information in the medication database 101, the processor 11 obtains the total number (for example, 10) of users (also known as second users) using the exact same medicines A, B, and C or the completely different medicines D, E, and F on the date of using the medicines A, B, and C in sequence among the first users. The processor 11 may record the medication route information of the second user on these dates (i.e., the second medication route information) in the medication database 101.

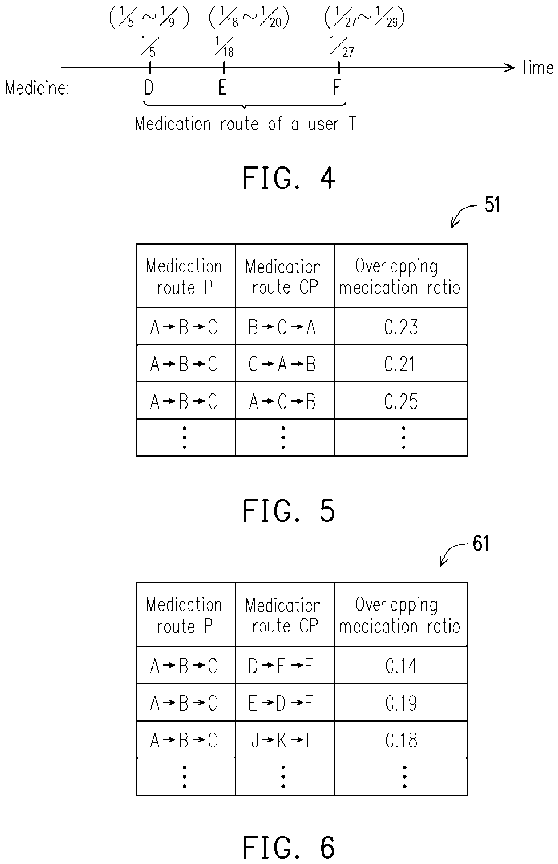

FIG. 4 is a schematic diagram of medication route information of a second user according to an embodiment of the present disclosure. In FIG. 4, which follows the embodiment of FIG. 3, assuming that among the first users, a certain user (for example, a user T), besides using the medicines A, B, and C on January 5, January 18, and January 27, also uses the medicines D, E, and F in sequence on January 5, January 18, and January 27. The processor 11 may mark the user T as the second user, and record the medication route information of the user T (i.e., the second medication route information) in the medication database 101.

Note that, in one embodiment, the definition of the second user may be broader. For example, in one embodiment in which the user X is taken as an example, assuming that the doses of the medicines A, B, and C are respectively for 3 days, 5 days, and 3 days, then the processor 11 broadens the date of using the medicines A, B, and C to respectively fall into the periods of: April 17 to April 19, April 22 to April 26, and May 1 to May 3. Or, taking the user T as an example, and assuming that the doses of the medicines A, B, and C are respectively for 5 days, 3 days, and 3 days, then the processor 11 broadens the dates of using the medicines A, B, and C to respectively fall into the periods of: January 5 to January 9, January 18 to January 20, and January 27 to January 29. Therefore, in an embodiment of FIG. 3, assuming that the user X, besides using the medicines A, B, and C in sequence on April 17, April 22, and May 1 (or April 17 to April 19, April 22 to April 26, and May 1 to May 3), also uses the medicines D, E, and F in sequence on April 17 to April 19, April 22 to April 26, and May 1 to May 3, then the user X meets the definition of the second user. Similarly, in an embodiment of FIG. 4, at the time of January 5 to January 9, January 18 to January 20, and January 27 to January 29, assuming that the user T, while using the medicines A, B, and C in sequence, also uses the medicines D, E, and F in sequence, then the user Y also meets the definition of the second user.

In one embodiment, the processor 11 determines the number of medication users that meet the first medication route information based on the total number of the first users, and determines the number of medication users that meet the second medication route information based on the total number of the second users. For example, assuming that the total number of the first users is 50, and the total number of the second user is 10, then the processor 11 determines that the number of medication users that meet the first medication route information is 50 and the number of medication users that meet the second medication route information is 10.

In one embodiment, according to the number of medication users that meet the information of the first medication route and the number of medication users that meet the information of the second medication route, the processor 11 determines the ratio value to reflect the degree of overlap between the number of medication users that meet the first medication route information and the number of medication users that meet the second medication route information. For example, assuming that the number of medication users that meet the first medication route information is 50 and the number of medication users that meet the second medication route information is 10, the processor 11 determines the ratio value to be 0.2 (0.2=10/50).

In an embodiment, assuming that the threshold value is 0.1, the processor 11 determines that the mentioned ratio value is greater than the threshold value. Therefore, the processor 11 may determine that the first medication route information meets the noise exclusion condition. Subsequently, this first medication route information will not be used to establish, train, or update the risk evaluation model 102. Or, in an embodiment, assuming that the threshold value is 0.3, the processor 11 determines that the ratio value is not greater than the threshold value. Then, the processor 11 may determine that the first medication route information does not meet the noise exclusion condition. Subsequently, this first medication route information will be used to establish, train, or update the risk evaluation model 102.

FIG. 5 is a schematic diagram of information based on first medication route information, second medication route information, and overlapping medication information according to an embodiment of the present disclosure. In FIG. 5, in this embodiment, it is assumed that the medicines in the first medicine combination are exactly the same as the medicines in the second medicine combination. The processor 11 may record the first medication route information (i.e., a medication route P), the second medication route information (i.e., a medication route CP), and the corresponding overlapping medication information (i.e., an overlapping medication ratio) in an information table 51.

In this embodiment, it is assumed that the medication route P is that the medicines A, B, and C are used in sequence on specific dates (for example, the dates mentioned in the embodiment of FIG. 3 and FIG. 4), and the medication route CP is that the medicines A, B, and C are also used on the same dates (for example, the dates mentioned in the embodiment of FIG. 3 and FIG. 4). However, note that the order of using the medicines A, B, and C in the medication route CP is different from the order of using the medicines A, B, and C in the medication route P.

Take the first row in the information table 51 as an example; the medication route P is to use the medicines A, B, and C in sequence, while the medication route CP is to use the medicines B, C, and A in sequence, and the degree of overlap between the number of medication users of the medication route P and the medication route CP may be represented by the ratio 0.23. That is to say, in this embodiment, assuming that the total number of the first users meeting the medication route P is 100, the total number of the second users meeting the medication routes P and CP at the same time is approximately 23, which accounts for a ratio of 0.23. The information in each row of the information table 51 may be created and filled in by analogy.

In one embodiment, it is assumed that the threshold value is 0.24. When screening out the medication routes to be excluded, medication route information related to the medication route P having a ratio value greater than 0.24 may be excluded. Take FIG. 5 as an example; the ratio between the medication route CP (A->C->B) and the medication route P (A->B->C) is greater than the threshold value 0.24, indicating that among users who have used the medicines A, B, and C, the proportion of those who use the medicines A, B, and C alternately is very high, and that the order in which the users use the medicines A, B, and C is quite inconsistent. Therefore, even if some users are hospitalized due to the use of the medicines A, B, and C, it may not be determined with certainty which order of using the medicines A, B, and C has a more significant impact on the user's hospitalization.

FIG. 6 is a schematic diagram of information based on first medication route information, second medication route information, and overlapping medication information according to an embodiment of the present disclosure. In FIG. 6, in this embodiment, it is assumed that the medicines in the first medicine combination are completely different from the medicines in the second medicine combination. The processor 11 may record the first medication route information (i.e., the medication route P), the second medication route information (i.e., the medication route CP), and the corresponding overlapping medication information (i.e., the overlapping medication ratio) in the information table 61.

In this embodiment, it is assumed that the medication route P is to use the medicines A, B, and C in sequence on specific dates (for example, the dates mentioned in the embodiment of FIG. 3 and FIG. 4), and the medication route CP is to use the medicines D, E, and F (or medicines J, K, and L) are further used on the same dates (for example, the dates mentioned in the embodiment of FIG. 3 and FIG. 4). The medicines A, B, and C are completely different from medicines D, E, and F (or the medicines J, K, and L).

Take the first row of the information table 61 as an example; the medication route P is to use the medicines A, B, and C in sequence, and the medication route CP is for the users who meet the medication route P that also use the medications D, E, and F in sequence. The degree of overlap between the numbers of medication users of the medication route P and the medication route CP may be represented by the ratio 0.14. That is to say, in this embodiment, assuming that the total number of the first users meeting the medication route P is 100, the total number of the second users meeting the medication routes P and CP at the same time is approximately 14, which accounts for a ratio of 0.14. The information in each row of the information table 61 may be created and filled in by analogy.

In an embodiment, it is assumed that the threshold value is 0.18. When screening out the medication routes to be excluded, medication route information related to the medication route P having a ratio value greater than 0.18 may be excluded. Take FIG. 6 as an example; the ratio between the medication route CP (E->D->F) and the medication route P (A->B->C) is 0.19 is greater than the threshold value 0.18, indicating that among the users who have used the medicines A, B, and C, there are more than 18 % of the users who are also using the medicines D, E, and F at the same time. Therefore, even if some users are hospitalized due to the use of the medicines A, B, and C and the medicines D, E, and F, it may not be determined with certainty what medicines in and/or which order of using the medicines A, B, and C and the medicines D, E, and F have a more significant impact on the user's hospitalization. Therefore, in one embodiment, the order of using the medicines A, B, and C (i.e., the medication route P(A->B->C)) may be treated as noise for the risk evaluation model 102 and is thus excluded by the processor 11 (that is, it is not used to establish, train, or update the risk evaluation model 102).

Note that in another embodiment of FIG. 5 and/or FIG. 6, if the ratio values related to the medication route P are not greater than the threshold value (for example, 0.4), the processor 11 does not exclude medication route information related to the medication route P when subsequently establishing or updating the risk evaluation model 102 (that is, the medication route information related to the medication route P may be adapted to establish, train, or update the risk evaluation model 102).

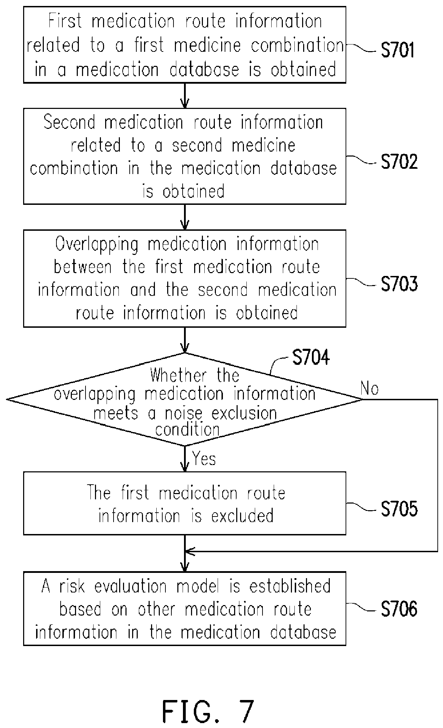

FIG. 7 is a flowchart of a medication risk evaluation method according to an embodiment of the present disclosure. In FIG. 7, in step S701, first medication route information related to a first medicine combination in a medication database is obtained. In step S702, second medication route information related to a second medicine combination in the medication database is obtained. In step S703, overlapping medication information between the first medication route information and the second medication route information is obtained. In step S704, it is determined whether the overlapping medication information meets a noise exclusion condition. If the overlapping medication information meets the noise exclusion condition, then in step S705, the first medication route information is excluded, and in step S706, a risk evaluation model is established based on other medication route information in the medication database (that is, the other medication route information that does not include the first medication route information), where the risk evaluation model is adapted to evaluate the risk of using at least one medicine in the medication database. Or, in step S704, if it is determined that the overlapping medication information does not meet the noise exclusion condition, then step S705 is skipped and step S706 is performed.

As each step in FIG. 7 has been described in detail as above, they are not repeated here. It is worth noting that each step in FIG. 7 may be implemented as multiple program codes or circuits, and the present disclosure is not limited thereto. In addition, the method in FIG. 7 may be adapted in conjunction with the above embodiments, or may be used alone, and the present disclosure is not limited thereto.

In summary, the medication risk evaluation method and the medication risk evaluation device provided by the embodiments of the present disclosure are capable of excluding the medication route information that may cause noise or uncertainty in the process of establishing, training, or updating the medication risk evaluation model, thereby improving the reliability of the established risk evaluation model.

Although the present disclosure has been disclosed in the above embodiments, it is not intended to limit the present disclosure. Anyone with ordinary knowledge in the relevant technical field can make changes and modifications without departing from the spirit and scope of the present disclosure. Therefore, the scope of this disclosure shall be in accordance with the scope of the patent claims as attached.

### [Descriptions of Reference Numerals]

10: medication risk evaluation device
11: processor
12: storage circuit
101: medication database
102: risk evaluation model
51, 61: information table
S701 to S706: steps

## Claims

1. A medication risk evaluation method, comprising:
obtaining first medication route information related to a first medicine combination in a medication database (101);
obtaining second medication route information related to a second medicine combination in the medication database (101);
obtaining overlapping medication information between the first medication route information and the second medication route information;
determining whether the overlapping medication information meets a noise exclusion condition; and
if the overlapping medication information meets the noise exclusion condition, establishing a risk evaluation model (102) based on other medication route information in the medication database (101) excluding the first medication route information,
wherein the risk evaluation model (102) is adapted to evaluate a risk of using at least one medicine in the medication database (101).

2. The medication risk evaluation method according to claim 1, wherein medicines in the first medicine combination are exactly the same as medicines in the second medicine combination.

3. The medication risk evaluation method according to claim 1 or 2, wherein medicines in the first medicine combination are completely different from medicines in the second medicine combination.

4. The medication risk evaluation method according to one of the preceding claims, wherein the overlapping medication information reflects a degree of overlap between a number of medication users that meet the first medication route information and a number of medication users that meet the second medication route information.

5. The medication risk evaluation method according to claim 4, wherein determining whether the overlapping medication information meets the noise exclusion condition comprises:
obtaining a ratio value according to the overlapping medication information, wherein the ratio value reflects the degree of overlap;
determining whether the ratio value is greater than a threshold value;
if the ratio value is greater than the threshold value, determining that the overlapping medication information meets the noise exclusion condition; and
if the ratio value is not greater than the threshold value, determining that the overlapping medication information does not meet the noise exclusion condition.

6. A medication risk evaluation device (10), comprising:
a storage circuit (12), adapted to store a medication database (101); and
a processor (11), coupled to the storage circuit (12), wherein
the processor (11) is adapted to:
obtain first medication route information related to a first medicine combination in the medication database (101);
obtain second medication route information related to a second medicine combination in the medication database (101);
obtain overlapping medication information between the first medication route information and the second medication route information;
determine whether the overlapping medication information meets a noise exclusion condition; and
if the overlapping medication information meets the noise exclusion condition, establish a risk evaluation model (102) based on other medication route information in the medication database (101) excluding the first medication route information, wherein
the risk evaluation model (102) is adapted to evaluate a risk of using at least one medicine in the medication database (101).

7. The medication risk evaluation device (10) according to claim 6, wherein medications in the first medicine combination is exactly the same as medications in the second medicine combination.

8. The medication risk evaluation device (10) according to claim 6, wherein medications in the first medicine combination is completely different from medications in the second medicine combination.

9. The medication risk evaluation device (10) according to claim 6, wherein the overlapping medication information reflects a degree of overlap between a number of medication users that meet the first medication route information and a number of medication users that meet the second medication route information.

10. The medication risk evaluation device (10) according to claim 9, wherein an operation of determining whether the overlapping medication information meets the noise exclusion condition comprises:
obtaining a ratio value according to the overlapping medication information, wherein the ratio value reflects the degree of overlap;
determining whether the ratio value is greater than a threshold value;
if the ratio value is greater than the threshold value, determining that the overlapping medication information meets the noise exclusion condition; and
if the ratio value is not greater than the threshold value, determining that the overlapping medication information does not meet the noise exclusion condition.
